# EUROPÄISCHE PATENTSCHRIFT

(11) **EP 2 617 439 B1**
(45) Veröffentlichungstag und Bekanntmachung des Hinweises auf die Patenterteilung: **27.02.2019**
(21) Anmeldenummer: 13151346.7
(22) Anmeldetag: 15.01.2013
(51) Int. Cl.: A61L 27/32, A61L 27/54, A61L 27/56

(54) **ANTIBAKTERIELLE UND OSTEOINDUKTIVE IMPLANTATBESCHICHTUNG, VERFAHREN ZUR HERSTELLUNG EINER SOLCHEN BESCHICHTUNG SOWIE DAMIT BESCHICHTETES IMPLANTAT**
ANTIBACTERIAL AND OSTEOINDUCTIVE IMPLANT COATING, METHOD FOR PRODUCING SUCH A COATING AND IMPLANT COATED WITH THE SAME
REVÊTEMENT D'IMPLANT ANTIBACTÉRIEN ET OSTÉO-INDUCTEUR, PROCÉDÉ DE FABRICATION D'UN TEL REVÊTEMENT ET IMPLANT REVÊTU AINSI

(30) Priorität: 23.01.2012 DE 102012001260
(43) Veröffentlichungstag der Anmeldung: 24.07.2013
(73) Patentinhaber: DOT GmbH, 18059 Rostock (DE)
(72) Erfinder: NEUMANN, Hans-Georg, 18146 Rostock (DE); PRINZ, Cornelia, 18119 Rostock (DE)
(74) Vertreter: Prinz & Partner mbB

(56) Entgegenhaltungen:
- WO-A1-02/05862
- WO-A2-2009/062671

## Beschreibung

Die vorliegende Erfindung betrifft eine antibakterielle und zugleich osteoinduktiv wirkende Beschichtung für Implantate, insbesondere für metallische bzw. für eine elektrochemische Beschichtung ausreichend leitfähige Implantate, ein Verfahren zur Herstellung einer derartigen Beschichtung sowie ein Implantat mit einer solchen Beschichtung.

Aus dem Stand der Technik ist bereits bekannt, dass Implantatoberflächen zur optimalen Integration der Implantate in das umgebende Körpergewebe modifiziert werden müssen. Dies erfordert, dass diese Oberflächen bioaktiv sind. Das heißt, dass Gewebe oder Zellen spezifisch, nämlich in Hinblick auf ihre Differenzierung und Proliferation, durch die Implantatoberfläche so beeinflusst werden, dass sie einen dauerhaften, kraftschlüssigen Verbund mit der Implantatoberfläche eingehen.

Die ebenfalls maßgeblich durch die Merkmale der Oberfläche bestimmte Biokompatibilität von Implantaten ist durch die Forschungsbemühungen der letzten Jahre entscheidend verbessert worden. In vielen Fällen müssen diese Oberflächeneigenschaften unabhängig von den Eigenschaften des Implantatwerkstoffs gestaltet werden, beispielsweise durch eine Beschichtung (Prinz, C: Funktionalisierung von Implantatoberflächen. Dissertation, Universität Rostock, 2009).

Weltweit werden jährlich ca. eine halbe Million Hüftendoprothesen implantiert. Dabei gehört eine implantatinduzierte Infektion nach wie vor zu den am meisten gefürchteten Komplikationen (van Osten, U., Salito, A., Breme, F., Aits, M., Hufnagel, K., 2008: http://www.gfe-online.de/opencms2/export/sites/default/PDF/ Veroeffentlichungen/Max-schneidern_porxserx4FEE2.pdf, 15.11.2008) und stellt die Hauptursache für Revisionsoperationen dar. Es ist daher von großer Bedeutung, Implantatoberflächen so zu gestalten, dass sie nicht nur zellstimulierend, sondern auch antibakteriell wirken.

Als Stand der Technik zur Beschichtung von Implantaten galt seit den 80er Jahren die Kombination aus plasmagespritztem Titan (TPS) und der schwerlöslichen Calciumphosphatphase Hydroxylapatit (HA). Calciumphosphate werden dabei verwendet, da sie als mineralische Bestandteile des Knochengewebes bioaktive Eigenschaften haben, d. h. sie unterstützen die Knochenbildung. Das Hydroxylapatit wird mit einer Dicke von >50-200 µm ebenfalls im Plasmaspritz-Verfahren auf die Implantatoberflächen aufgebracht.

Als nachteilig hat sich dabei herausgestellt, dass das HA-Pulver beim Spritzprozess thermisch zerfällt, was u. a. lokal unterschiedliche Löslichkeiten zur Folge hat und zur Unterwanderung bzw. Ablösung der Beschichtung führen kann.

Damit ist gemeint, dass durch das Plasmaspritzen thermische Einflüsse die Porosität und somit die Schichtqualität derart beeinflussen, dass die aufgetragene Schicht monolithisch ist. Die geringe Porosität führt zu der Schwerlöslichkeit der plasmagespritzten Calciumphosphatphase. Ferner sind komplexe Geometrien mittels Plasmaspritzen schwer zu beschichten.

Beispielsweise kann amorphes Calciumphosphat wegen seiner sehr hohen Löslichkeit in vivo zu Schichtdelaminationen und Abplatzungen führen. Als Folge solcher Schichtdelaminationen kann es zur Bildung einer Bindegewebskapsel im entstandenen Spalt kommen, was letztendlich in einer aseptischen Lockerung des Implantates resultieren würde (siehe Heimann R.: Entwicklung biokeramischer Beschichtungen für Hüftendoprothesen Teil 2; www.tu-clausthal.de/presse/ tucontact/2005/Mai/pdf).

Daher werden seit etwa 20 Jahren elektrochemisch abgeschiedene Calciumphosphat-Schichten auf Knochenimplantaten mit der Zielsetzung verwendet, den Verbund zwischen Knochengewebe und Implantat zu verbessern. Dies ist beispielsweise aus US 5,205,921, US 5,310,464, US 2008/0261034, EP 774 982 A1, US 5,759,376, DE 195 04 386, EP 1 301 220 A1, Redepenning, J. et al.: Chem. Mater. 2 (1990) 625-7, und Kumar, M. et al.: J. Biomed. Mat. Res. 45 [1999] 302-10, bekannt. Aus elektrolytischen Bädern auf Implantaten abgeschiedene Calciumphosphat-Schichten sind in ihrer Genese den beim Knochenwachstum ablaufenden Prozessen sehr ähnlich. Ihre mikroporöse Struktur kann erhalten bleiben, da keine thermischen Einflüsse auftreten. Die mikroporöse Struktur begünstigt die Immobilisierung von Zellen, von denen ausgehend sich neues Knochengewebe bilden und mit dem wie bei der normalen Wundheilung das Implantat zusammenwachsen kann.

Zudem ist aus der Literatur seit einigen Jahren bekannt, dass der Grad der Bioaktivität einer Schicht mit ihrer Instabilität in physiologischer Umgebung wächst. Damit ist gemeint, dass nach heutiger Erkenntnis die bioaktiven Beschichtungen nur so lange auf den Implantatoberflächen vorhanden sein müssen, bis die Osteointegration des Implantats erreicht ist, also ein struktureller Verbund hergestellt ist. Durch die lokale Erhöhung an Calcium- und Phosphationen-Konzentration in der Einheilzone des Implantats werden gute Bedingungen für die Vermehrung und Differenzierung der für die Knochenbildung verantwortlichen Osteoblasten geschaffen (Ducheyne, P. et al.: Biomaterials 11(1990) 531-40). Calciumphosphate mit einer im Vergleich zum Hydroxylapatit sehr viel höheren Löslichkeit wie Bruschit und Monetit haben somit speziell für die Einheilungsphase des Implantats in den Knochen eine besondere Bedeutung, da sie aufgrund der höheren Porosität eine höhere Löslichkeit aufweisen, wodurch die Konzentration der Calcium- und Phosphationen schnell ansteigt.

Wie bereits eingangs erwähnt, stellt eine implantatinduzierte Infektion mit Bakterien die am meisten gefürchtete Komplikation dar. Daher besteht ein besonders großes Interesse daran, die Implantatoberflächen derart auszuführen, dass sie zudem antibakteriell wirken.

In der Literatur werden bereits vielfach die bakterienhemmenden Eigenschaften von Kupfer beschrieben, wie beispielsweise bei Hubacher et al. N Engl J Med 345 (2001) 561-7 sowie in der zuvor genannten Dissertation von Prinz. Seit einiger Zeit ist bekannt, dass Kupfer neben seiner die Angiogenese fördernden Wirkung auch in freier, nicht an Proteine gebundener, Form antibakteriell wirkt. Man spricht hier, ähnlich wie beim Silber, vom oligodynamischen Effekt. Obwohl der Wirkmechanismus noch nicht vollständig geklärt ist, gilt als sicher, dass Metallkationen die Zellen an verschiedenen Stellen attackieren und so physiologische Funktionen, wie z. B. die Zellmembran-, die RNA- und die DNA-Synthese, die Translation oder die Proteinsynthese inaktivieren. Die Kupferionen sollen die Anhaftung und Vermehrung von klinischen Keimen wie Staphylococcus aureus auf der Implantatoberfläche unterbinden sowie mit definierter Freisetzungsrate deren Vermehrung im umgebenden Körpergewebe verhindern.

Der große Vorteil der Kupferionen im Gegensatz zu Antibiotika besteht darin, dass Bakterien bei abnehmenden Kupfer-Konzentrationen keine Resistenzen ausbilden, die antibakterielle Wirkung aber schon bei Konzentrationen erreicht wird, die noch nicht zellschädigend sind (Egler, M. "Rolle von RpoE-homologen Sigmafaktoren in der Schwermetall-Homöostase von Escherichia coli und Cupriavidus metallidurans", Dissertation, Martin-Luther-Universität Halle-Wittenberg 2005). Kupferionen sind für den Stoffwechsel der Zellen von essenzieller Bedeutung und in den Körpermedien mit Konzentrationen von ca. 1 mg/l vorhanden. Eine lokale Erhöhung dieser Konzentration auf das Zehnfache ist ausreichend, um eine deutliche antibakterielle Wirkung zu erzielen. Dadurch bietet sich durch eine antiinfektiöse Oberflächenmodifizierung von Knochenimplantaten mit Kupfer die Chance, die in vorbereiteten Implantatlager vorhandenen Bakterien in ihrer Entwicklung zu hemmen, ohne die körpereigenen Gewebezellen nennenswert zu schädigen bzw. in ihrer Entwicklung zu beeinträchtigen (zuvor genannte Veröffentlichung von Hubacher et al.).

Untersuchungen zum Einfluss von Kupfer auf die Zellzahl verglichen mit den Untersuchungen zur Zellviabilität zeigen, dass die Anzahl der Zellen durch das freigesetzte Kupfer zwar reduziert, die Zellviabilität jedoch nicht entscheidend gestört wird (Dissertation von Prinz sowie Suska, F., Esposito, M., Gretzer, C., Källtorp, M., Tengvall, P., Thomsen, P. (2003): IL-1α, IL-1β and TNF-α secretion during in vivo/ex vivo cellular interactions with titanium and copper Biomaterials, Volume 24, Issue 9, April 2003, Page 1683). Für die Toxizität bzw. die biologische Rückantwort sind also die chemischen Eigenschaften der Metallionen, ihre Zusammensetzung sowie die Konzentration im Gewebe als Funktion der Zeit von großer Bedeutung (Suska, F. (2004): "On the inflammatory response to variations in biomaterial surface chemistry"; ISBN 91-628-6302-9).

Inwieweit Kupfer einen Einfluss auf die HIF-1-Aktivierung und somit auf die Gefäßneubildung besitzt, ist bisher wenig untersucht. Bekannt sind aber ein die Angiogenese fördernder Effekt und eine VEGF-Induktion durch Kupfer (Parke, A., Bhattacherjee, P., Palmer, R.M., Lazarus, N.R., 1988: "Characterization and quantification of copper sulfateinduced vascularization of the rabbit cornea", Am J Pathol 130, 173-178; sowie Sen et al. (2002): "Copper-induced vascular endothelial growth factor expression and wound-healing", Am J Physiol Heart Circ Physiol 282, H1821-1827).

Aus der Literatur ist zudem bekannt, dass Kupfer positiv bei Wundheilungsprozessen wirkt. Borkow stellte in diesem Zusammenhang fest, dass GHK-Cu2+ antiinflammatorisch wirkt und die Geweberegeneration fördert. GHK (Glycyl-L-histidyl-L-lysine) ist eine Aminosäure, die eine hohe Affinität gegenüber Kupfer aufweist. Er beobachtete weiterhin, dass durch Cu2+-Ionen die Angiogenese sowie die Endothelzellproliferation stimuliert werden (Borkow, G. (2004): "Copper's Role in Wound Healing", Review of Literature, Property of Cupron Inc (CUPRON Better Health Through TechnologyTM) http://www.pedorthicnewswire.com/.../Copper%20Role%20in%20Wound%20Heali ng.pdf 12.04.2008). Eine hinreichende Kupferversorgung scheint für die Funktion von Zytokinen, die bei der Angiogenese, der Fibrinfaserbildung bei Entzündungen sowie bei der Wundheilung eine Rolle spielen, notwendig zu sein (zuvor genannte Veröffentlichung von Sen et al. sowie Parke et al. (1988): "Characterization and quantification of copper sulfate-induced vascularization of the rabbit cornea", Am J Pathol 130, 173-178).

Anwesende Bakterien nehmen dabei genau so viel Kupfer auf, bis eine für sie toxische Dosis erreicht ist. Vilchez et al. (Vilchez et al. (2007): "Dominance of sphingomonas in a copper exposed biofilm community for groundwater treatment" Microbiology 153 (Pt 2) 325-37, http://www.ncbi.nlm.nih.gov/pubmed/17259604) beschreiben, dass dieses Phänomen zur Entfernung von Kupfer aus dem Grundwasser verwendet werden kann. Die Autoren bezeichnen diesen Prozess als Bioakkumulation, bei dem es sowohl auf das Metallion als auch auf die Mikroorganismen ankommt. Dieser Mechanismus wird allein dadurch begrenzt, dass jeweils neue Kupferionen zur Verfügung stehen müssen. Dies lässt die Schlussfolgerung zu, dass eine Neuinfektion mit Bakterien verhindert wird, solange ausreichend Kupfer aus einer Oberfläche an das umgebende Gewebe abgegeben werden kann.

Ein Beispiel für eine Beschichtung, die mittels thermischen Spritzverfahrens aufgebracht wird, ist in der WO 2009/062671 gezeigt. Es wird ein Verfahren angegeben, mit dem ein Implantat im Verankerungsbereich mit einer Deckschicht als Beschichtung, bestehend aus Calciumphosphat und antibakteriell wirksamen Wirkbestandteilen, beschichtet wird. Diese Deckschicht wird unter Anwendung eines thermischen Spritzverfahrens aufgebracht, was eine monolitische Struktur hervorbringt. Das Calciumphosphat-Spritzpulver ist von körniger Beschaffenheit und wird mit Metallkörnern gemischt. Der Calciumphosphatanteil besteht vorzugsweise aus Hydroxylapatit, für die antibakteriell wirksamen Bestandteile werden Silber und/oder Kupfer bevorzugt.

Aus der WO 02/05862 A ist zudem ein Verfahren zur Herstellung einer Beschichtung bekannt, bei der Calciumphosphat-Schichten elektrochemisch abgeschieden werden.

Ferner hat sich gezeigt, dass Zell- und Bakterienadhäsionsprozesse stark von der Oberflächenbeschaffenheit abhängig sind. Vor allem die chemische Zusammensetzung, die hygroskopischen Eigenschaften wie auch die Rauigkeit der Oberfläche spielen dabei eine nicht zu vernachlässigende Rolle. Bei der Adhäsion von Bakterien besteht ein Zusammenhang zwischen ihrer Größe und Form und der Oberflächentopographie. Carolin Diaz et al. (Diaz et al.: "Influence of the Nano-micro Structure of the Surface on Bacterial Adhesion"; Materials Research 10, No. 1, (2007) 11-14) beschreiben, dass ein Anwachsen der Bakterien begünstigt wird, wenn die topologischen Eigenschaften der Oberfläche der Bakterienform und -größe entsprechen. Sie untersuchten glatte und raue Kupfer- und Goldschichten und fanden heraus, dass die Biofilmbildung auf den rauen Kupferoberflächen kaum oder gar nicht möglich war. Die hohe Bioaktivität, bei gleichzeitig antibakterieller Wirksamkeit, dieses schwammartigen Komposits äußert sich in der hervorragenden Benetzung durch die im Wundbett des Knochens befindliche Körperflüssigkeit und der damit verbundenen Adhäsion der in dieser Flüssigkeit enthaltenen, die Osteogenese stimulierenden, Faktoren.

Der Erfindung liegt die Aufgabe zugrunde, eine Beschichtung zu schaffen, deren antibakterielle Wirksamkeit verbessert ist, und somit die Verbindung eines mit dieser Beschichtung versehenen Implantats mit dem Knochen zu verbessern.

Erfindungsgemäß wird diese Aufgabe durch die Merkmale des Anspruchs 1 gelöst. Vorteilhafte Ausgestaltungen ergeben sich aus den Unteransprüchen.

Ziel der Erfindung ist es, eine Beschichtung bereitzustellen, die antibakteriell wirksame Bestandteile wie Kupferionen aufweist, die mit definierter Freisetzungsrate aus der Implantatoberfläche in das Körpergewebe wandern und dort die Vermehrung von klinischen Keimen wie Staphylococcus aureus verhindern. Die auf der Oberfläche des Knochenimplantats abgeschiedene Schicht soll zudem die Einheilung des Implantats in den Knochen optimal unterstützen, dabei völlig resorbiert werden und damit den direkten und vollständigen kraftschlüssigen Kontakt des Knochens mit dem Implantat ermöglichen. Dazu ist es von Vorteil, wenn die Calciumphosphat-Schicht hochporös ist, da dies eine hohe Löslichkeit garantiert. Das nachträgliche Einbringen der Kupferionen in die hochporöse Calciumphosphat-Schicht gewährleistet, dass sich die Kupferionen an den Schwachstellen der Calciumphosphat-Schicht anlagern. Damit kann die definierte Freisetzungsrate der Kupferionen erreicht werden, da diese diskontinuierlich und inhomogen in der zuvor aufgebrachten Calciumphosphat-Schicht verteilt sind.

Vorzugsweise ist vorgesehen, dass diese Schicht eine hochporöse Struktur aufweist, die es erlaubt, die Schicht sowohl während der Abscheidung als auch durch eine Nachbehandlung hinsichtlich ihrer Phasenzusammensetzung und Löslichkeit kontrolliert zu modifizieren und somit eine gesteuerte Resorption dieser Schicht möglich ist. Damit kann die Beschichtung auf die Erfordernisse des Einsatzortes des Implantats eingestellt werden. Die Porosität der Calciumphosphat-Schicht geht dabei mit der Löslichkeit dieser Schicht einher, wobei die Löslichkeit für die Verbindung des Implantats mit dem Knochengewebe ein wichtiger Parameter ist.

Insbesondere ist diese Schicht derart ausgebildet, dass sie Kupferionen eluiert, die die Angiogenese des angrenzenden Gewebes fördern. Die Kompositschicht enthält neben den Calciumphosphat-Phasen zusätzlich Kupferphosphat-Phasen und Kupfer. Aus dieser Kompositschicht eluieren neben den Kupferionen demnach auch die das Wachstum der Zellen stimulierenden Calcium- und Phosphationen. Durch die hochporöse Calciumphosphat-Phase wird das Anhaften der Zellen begünstigt. Die Ausbildung eines bakteriellen Biofilms wird durch die Elution der Kupferionen erschwert bzw. verhindert.

Die Kompositschicht kann in Aufbau, Zusammensetzung und Dicke an das jeweilige Implantat und den das Implantat umgebenden Knochen angepasst werden. Die Herstellung dieses Komposits kann dabei so vor sich gehen, dass die einzelnen Phasen gleichzeitig oder auch nacheinander abgeschieden bzw. Anteile der einen, z. B. der löslicheren Calciumphosphat-Phase, durch eine chemische Reaktion in eine weniger lösliche Phase überführt werden.

Insbesondere ist die Kupferkonzentration in der Beschichtung so gewählt, dass eine hohe Anfangselution durch die Auflösung elementaren Kupfers hinreichend antimikrobiell wirkt und moderate längerzeitige Auflösungen von Kupferphosphaten keine toxische Wirkung hervorruft. Die Kupfer enthaltenden Phasen gewährleisten über einen längeren Zeitraum eine bakterienfreie Einheilzone und begünstigen damit die Ausweitung und Stabilisierung des neuen Knochengewebes. Die hochporöse Calciumphosphat-Schicht gewährleistet die hohe Löslichkeit und die damit verbundene hohe Anfangselution des Kupfers.

Auf Grund der durch die Parameter der elektrochemischen Abscheidung einstellbaren hochporösen Struktur kann und soll die Kompositschicht nicht als Barriere zwischen Implantat und Knochen, sondern soll als harmonisierender Faktor oder Wirt bei der körpereigenen Strukturierung der Grenzschicht zwischen Implantat und Knochen dienen. Zell- und Bakterienadhäsionsprozesse sind stark von der Oberflächenbeschaffenheit abhängig. Vor allem die chemische Zusammensetzung, die hygroskopischen Eigenschaften wie auch die Rauigkeit der Oberfläche spielen dabei eine nicht zu vernachlässigende Rolle.

In einer bevorzugten Ausführungsform ist vorgesehen, dass die Schicht Kupfer in einer solchen Rate freisetzen kann, dass sich in einer das Implantat umgebenden Körperflüssigkeit eine Kupferkonzentration von 90 bis 160 µmol/l ergibt. Der Anteil an Kupfer in der Beschichtung ist ausreichend hoch, um eine antimikrobielle Wirkung zu entfalten. Die Konzentration der im Körper freigesetzten Kupferionen ist jedoch nicht so hoch, dass es zu einer zellschädigenden Wirkung und damit zu einer Beeinträchtigung des Anwachsens des Implantats kommt. Diese Kupferkonzentration gewährleistet demnach, dass sich die antimikrobielle Wirkung entfalten kann, wobei noch keine toxischen Folgen für das das Implantat umgebende Zellgewebe eintritt.

Vorzugsweise beträgt die Schichtdicke 20 µm ± 10 µm. Diese geringe Schichtdicke ist ausreichend, um das Einwachsen des Implantats in das Zellgewebe zu garantieren, wobei dies aufgrund der hochporösen Struktur der Calciumphosphat-Schicht ermöglicht wird.

Ferner ist ein Implantat mit einer Beschichtung der zuvor genannten Art vorgesehen, welches aufgrund der aufgetragenen Beschichtung die zuvor genannten Vorteile hinsichtlich der Angiogenese aufweist.

Zudem ist ein Verfahren zur Herstellung einer Beschichtung der zuvor genannten Art vorgesehen, welches die folgenden Schritte umfasst: Bereitstellen eines Implantats; zunächst das Implantat mit Calciumphosphat beschichten, welches elektrochemisch abgeschieden wird; nachträglich Kupfer in die zuvor aufgebrachte Calciumphosphat-Schicht einbringen. Mit diesem Verfahren wird gewährleistet, dass das Kupfer diskontinuierlich und inhomogen in der hochporösen Calciumphosphat-Schicht verteilt ist, sodass eine definierte Freisetzungsrate der Kupferionen, die die antimikrobielle Wirkung aufweisen, gewährleistet ist. Durch das elektrochemische Abscheidungsverfahren können zudem komplexere Geometrien des Substrats beschichtet werden.

In einer besonders bevorzugten Ausführungsform ist vorgesehen, dass das Calciumphosphat abgeschieden wird, insbesondere elektrochemisch, während sich das Implantat in einem Ultraschallbad befindet. Der Ultraschall dient der Entfernung von zu schwach an der Implantatoberfläche gebundenen Calciumphosphat-Kristallen. Die dabei entstehenden Löcher in der Schicht werden umgehend neu beschichtet.

Insbesondere ist vorgesehen, dass das Kupfer galvanisch abgeschieden wird. Dies bietet den Vorteil, dass sich das Kupfer auch in mit der Calciumphosphat-Schicht beschichteten komplexen Geometrien des Substrats anlagern kann.

In einer besonders bevorzugten Ausführungsform ist vorgesehen, dass das Kupfer abgeschieden wird, während sich das Implantat in einem Ultraschallbad befindet. Die durch den Ultraschall erzeugten Lücken in der Calciumphosphat-Schicht werden dabei vorwiegend mit Kupfer aufgefüllt. Das Kupfer legt sich somit an den Schwachstellen der Calciumphosphat-Schicht an, die durch das Ultraschallbad zum Vorschein kommen. Zudem werden schlecht haftende Kupferionen durch das Ultraschallbad gelöst, wobei diese Stellen erneut mit Kupfer versehen werden können.

Vorzugsweise wird das Calciumphospat in einer Dicke von etwa 20 µm abgeschieden. Diese Dicke ist aufgrund der hochporösen Calciumphosphat-Schicht bereits ausreichend, um das Einwachsen zu gewährleisten.

Insbesondere wird das Kupfer mit einer Masse von etwa 1 µg/mm² abgeschieden. Diese Abscheidungsrate gewährleistet, dass die Kupferkonzentration in der Beschichtung so gewählt ist, dass eine ausreichend antimikrobielle Wirkung vorhanden ist, jedoch keine toxische Wirkung, die zu Schäden des umliegenden Gewebes führt, hervorgerufen werden.

Die Erfindung wird nachfolgend anhand von Abbildungen und eines Ausführungsbeispiels erläutert:
- Figur 1 zeigt eine rasterelektronenmikroskopische Aufnahme einer erfindungsgemäßen elektrochemisch abgeschiedenen Calciumphosphat (Bruschit)/Kupfer-Beschichtung,
- Figur 2 die Kupfer-Freisetzung aus einer erfindungsgemäßen Calciumphosphat/Kupfer(CaP/Cu)-Implantatbeschichtung in RPMI-Zellkulturmedium und
- Figur 3 das Verhalten von biofilmbildenden und planktonischen Staphylococcus aureus in RPMI-Zellkulturmedium über einer erfindungsgemäßen CaP/Cu-Implantatbeschichtung und über einer kupferfreien CaP-Beschichtung.

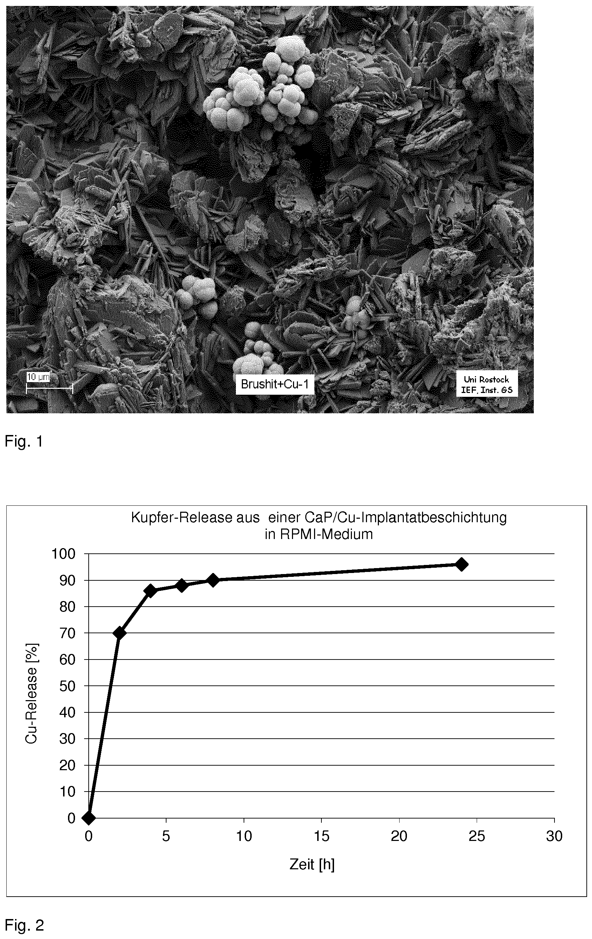

Figur 1 zeigt eine rasterelektronenmikroskopische Aufnahme einer erfindungsgemäßen Calciumphosphat/Kupfer-Beschichtung, die elektrochemisch abgeschieden worden ist, wobei Bruschit als hochporöse Calciumphosphat-Schicht verwendet worden ist, in das sich Kupfer diskontinuierlich eingelagert hat.

Die hochporöse Struktur der Calciumphosphat-Schicht ist in der Figur 1 schwer zu erkennen, da aufgrund der rasterelektronenmikroskopischen Aufnahme eine sehr detaillierte Aufnahme der Beschichtung gemacht worden ist. Dennoch sind die Calciumphosphat-Kristallite zu erkennen, die als Plättchen oder Nadeln nahezu senkrecht auf der Implantatoberfläche fixiert sind. Mit dieser mikroporösen bzw. hochporösen Calciumphosphat-Schicht wird eine große Reaktionsfläche für die Wechselwirkung mit der biologischen Umgebung geschaffen. Ferner sind Kupferionen zu erkennen, die sich in der hochporösen Calciumphosphat-Schicht diskontinuierlich und inhomogen verteilt eingelagert haben.

Vorzugsweise ist die Calciumphosphat-Schicht auf das Implantat mittels eines elektrochemischen Abscheidungsverfahrens aufgetragen worden, wobei das Kupfer ebenfalls galvanisch in die bestehende Calciumphosphat-Schicht eingelagert worden ist. Durch gezielte Variation der Beschichtungsparameter ist die Einstellung verschiedener Calcium-Phosphat-Verhältnisse und Porositäten entsprechend den Anforderungen einstellbar. Im Detail ergibt sich die Beschichtung aus einem elektrochemischen Prozess, bei dem zunächst durch Anlegen einer negativen Spannung an das metallische Implantat auf seiner stark basischen Oberfläche aus dem Elektrolyten eine etwa 15 µm dicke hochporöse und damit sehr bioaktive Calciumphosphat(Bruschit)-Schicht abgeschieden wird. Das Kupfer wird im Anschluss vorzugsweise aus einer gesättigten Kupferacetat-Lösung diskontinuierlich und inhomogen in der hochporösen Calciumphosphat-Schicht galvanisch eingelagert.

Die Calciumphosphat-Schicht sowie das Kupfer werden bevorzugt elektrochemisch eingelagert, wobei die Beschichtung während eines Ultraschallbades stattfindet. Das Ultraschallbad sorgt dafür, dass Schwachstellen der Beschichtung gelöst werden und so neu beschichtet werden können. Dies gilt sowohl bei der Grundbeschichtung des Implantats mit der Calciumphosphat-Schicht als auch bei dem nachträglichen Einbringen des Kupfers in die bestehende hochporöse Calciumphosphat-Schicht. Bei dem Einlagern des Kupfers wird des Weiteren somit sichergestellt, dass sich das Kupfer in Schwachstellen der Calciumphosphat-Schicht einlagert. Dies impliziert, dass sich das Kupfer diskontinuierlich und inhomogen in der Calciumphosphat-Schicht verteilt.

Figur 2 zeigt die Freisetzung des Kupfers aus einer erfindungsgemäßen Calciumphosphat/Kupferbeschichtung gegenüber der Zeit. Die Auflösung des diskontinuierlich verteilten Kupfers in einem RPMI-Zellkulturmedium erfolgt sehr schnell. Schon nach 8 Stunden sind 90 % des auf der Probenoberfläche abgeschiedenen Kupfers im RPMI-Medium nachzuweisen. Ein Teil der in Lösung gehenden Kupferionen bildet mit den aus der Calciumphosphat-Beschichtung eluierenden Phosphationen schwerer lösliche Kupferphosphate, die im Wesentlichen den geringeren Kupfereintrag in der nachfolgenden Zeit ausmachen. Wie bereits erwähnt, wird durch die Anwesenheit von Kupfer die Angiogenese gefördert sowie der Wundheilungsprozess positiv beeinflusst. Daher ist es von Vorteil, dass das Kupfer schnell abgeschieden wird, um seine Wirkung entsprechend zu entfalten.

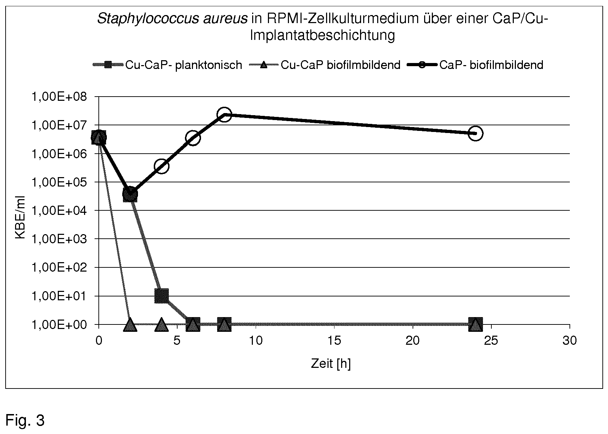

Figur 3 vergleicht das Verhalten von biofilmbildenden und planktonischen Staphylococcus aureus in RPMI-Zellkulturmedium einer erfindungsgemäßen Calciumphosphat/Kupferbeschichtung mit einer kupferfreien Calciumphosphat-Beschichtung. Die Figur 3 verdeutlicht somit die antibakterielle Wirkung des in die Calciumphosphat-Schicht eingebrachten Kupfers gegenüber einer Calciumphosphat-Schicht ohne Kupfereinlagerung. Das in Figur 3 gezeigte Diagramm zeigt die Bakterienkonzentration gegenüber der Zeit, wobei zur Untersuchung der antibakteriellen Wirkung der klinisch relevante Bakterienstamm Staphylococcus aureus ATCC25923 verwendet worden ist. Hierfür wurden Ti-Proben (Dicke = 1 mm, Durchmesser= 20 mm) mit Calciumphosphat und Kupfer (1µg/mm2) beschichtet und in 4 ml des mit 150 µl Bakteriensuspension beimpften RPMI-Zellkulturmediums bei einer Temperatur von 37 °C unterschiedlich lange ausgelagert. Danach wurde die Bakterienkonzentration auf den Probekörpern und in der Lösung durch Ausplattieren auf Nähragar und Auslagern bei 30 °C ermittelt. Als Referenz diente einer lediglich mit Calciumphosphat beschichtete Probenoberfläche. Auf dieser sind deutlich lebende Bakterien nachweisbar, auf der zusätzlich mit Kupfer beschichteten Oberfläche (CaP/Cu) dagegen nicht. Dies wird auch durch Laserlicht-Fluoreszenz-Mikroskopaufnahmen bestätigt. Bei den planktonischen Bakterien waren nach 6 Stunden keine vermehrungsfähigen Bakterien mehr nachzuweisen.

Die antibakterielle Wirkung des Kupfers ist somit eindeutig. Die rasche Abnahme der Bakterienkonzentration geht mit der sehr schnellen Kupferfreisetzung, die in Figur 2 gezeigt worden ist, einher, wobei sich das Kupfer aus der hochporösen Calciumphosphat-Schicht lösen kann, da das Kupfer diskontinuierlich und inhomogen in dieser hochporösen Schicht verteilt ist. Ferner ist die schnelle Freisetzungsrate des Kupfers damit zu begründen, dass sich das Kupfer vorwiegend in Schwachstellen der Calciumphosphat-Schicht eingelagert hat.

Die erfindungsgemäße kupferbeladene CaP-Schicht wurde hinsichtlich ihres Einflusses auf die Signaltransduktion bei der Gefäßneubildung und bei der Entzündung untersucht. Es konnte festgestellt werden, dass es keine Hinweise auf das Auftreten von Zelltod gibt. Das Anfärben des CD31-Proteins ließ sogar eine Aktivierung der Zellen durch Kupfer vermuten.

In den Figuren 1 bis 3 ist die antibakterielle Wirkung des Kupfers gezeigt worden sowie die Ursache für die rasche Freisetzung des Kupfers, die in der erfindungsgemäßen Ausgestaltung der Calciumphosphat/Kupfer-Beschichtung begründet ist. Im Weiteren soll eine bevorzugte Ausführungsform eines Substrats angegeben werden sowie dessen Beschichtung mit der erfindungsgemäßen Calciumphosphat/Kupfer-Beschichtung:
Eine geeignete Versuchsanordnung, um ein Substrat in der erfindungsgemäßen Weise zu beschichten, sieht wie folgt aus: Ein Becherglas mit 100 ml eines wässrigen Elektrolyten, enthaltend 0,1 mol/m NH4H2PO4 und 0,167 mol/l CaCl2, wird in ein Ultraschallbad gestellt. In dem Elektrolyten wird eine Titan-Probe (1 mm dick, 20 mm Durchmesser) als Kathode über ein Gleichstrom-Netzgerät mit einer Kohlenstoff-Anode verbunden. Bei moderatem Ultraschall wird bei 6 Volt in 10 Minuten eine 20 µm dicke hochporöse haftfeste Calciumphosphat-Schicht (ca. 20 µg/mm²) abgeschieden.

Auch beim Aufbringen des Kupfers ist es wie erwähnt vorteilhaft, das Substrat einem Ultraschallbad auszusetzen. Ein geeigneter Versuchsaufbau sieht dabei wie folgt aus: In einer gesättigten wässrigen Kupferacetat-Lösung wird die mit Calciumphosphat beschichtete Probe als Kathode über ein Gleichstrom-Netzgerät mit einer Kupferanode verbunden und 10 Sekunden bei 6 Volt mit Kupfer beschichtet. Dabei wird ca. 1 µg Kupfer/mm2 abgeschieden.

Somit ist eine erfindungsgemäße Calciumphosphat/Kupfer-Beschichtung auf einem Substrat aufgebracht worden, das aufgrund der Beschichtung eine verbesserte antibakterielle Wirksamkeit aufweist.

Ein derartig beschichtetes Implantat kann nun in einen menschlichen Körper implantiert werden, wobei nach der Implantation die folgenden Schritte vereinfacht durch drei Abschnitte charakterisiert werden können:
Im Abschnitt I werden zunächst die Kupferionen ausgeschwemmt. Bei Anwesenheit von Bakterien wird dieser Vorgang durch hohe Affinität der Bakterien zu Metallkationen beschleunigt. Dabei nehmen Bakterien genau so lange Kupfer auf, bis ihre letale Dosis erreicht ist. Die dafür erforderliche Zeit wird durch das Angebot an Kupfer bestimmt. Dies wurde in den Figuren 2 und 3 gezeigt und entsprechend erläutert. Der in der Literatur als "race for the surface" ("Wettlauf" zwischen den körpereigenen Zellen und den während der Operation eingebrachten Mikroorganismen) beschriebene Vorgang, der direkt nach dem Einbringen des Implantates stattfindet, wird aufgrund des Kupfers zu Gunsten der Zellen verschoben.

Im Abschnitt II wird die in der Grenzschicht zwischen Implantat und Knochen vorliegende größte lokale Erhöhung der Calcium- und Phosphationen-Konzentration im Wesentlichen durch die leichter löslichen Phasen bestimmt. In diesem Abschnitt muss gewährleistet werden, dass keine fibröse Einkapselung des Implantates eintritt und damit seine unmittelbare Osteointegration ermöglicht wird. Die Porosität des Komposits nimmt zunächst durch Abnahme der leichter löslichen Phasen zu, womit Platz für neues Knochengewebe geschaffen wird.

Im Abschnitt III wird der schwerer lösliche Kompositanteil aufgelöst. Die durch Anwesenheit von Kupferionen ausgefällten Kupferphosphate bestimmen den lokalen lonenhaushalt und unterstützen insbesondere die Mineralisierung und Angiogenese des neuen Knochengewebes. Nach Auflösen der weniger löslichen Calciumphosphat-Phasen ist das ganze Komposit in Knochengewebe umgewandelt.

Nach dem in Abschnitt III beschriebenen Auflösen der kompletten Beschichtung ist das gesamte Komposit durch neuen Knochen ersetzt und das Implantat ist kraftschlüssig in den Knochen eingeheilt.

Ausgehend von ersten Kontakten der immobilisierten Zellen mit dem Implantat über die Poren des Komposits hat sich durch die fortschreitende Auflösung der einzelnen Phasen von Anbeginn eine kraftschlüssige Verbindung zwischen Knochen und Implantat entwickelt.

Es ist somit eine Beschichtung für ein Implantat geschaffen, dessen antimikrobielle Wirkung verbessert ist und gleichzeitig den Einheilprozess beschleunigt.

## Patentansprüche

1. Calciumphosphat/Kupfer-Beschichtung für ein Implantat, **gekennzeichnet dadurch, dass** sie hochporöses Calciumphosphat und diskontinuierlich verteiltes Kupfer aufweist, wobei das hochporöse Calciumphosphat zunächst eine hochporöse Calciumphosphat-Schicht bildet, in die das Kupfer nachträglich diskontinuierlich und inhomogen verteilt eingelagert worden ist, um die Calciumphosphat/Kupfer-Beschichtung zu bilden.

2. Calciumphosphat/Kupfer-Beschichtung nach Anspruch 1, **gekennzeichnet dadurch, dass** die Calciumphosphat-Schicht eine hochporöse Struktur aufweist, die es erlaubt die Schicht sowohl während der Abscheidung als auch durch eine Nachbehandlung hinsichtlich ihrer Phasenzusammensetzung und Löslichkeit kontrolliert zu modifizieren und somit eine gesteuerte Resorption dieser Schicht möglich ist.

3. Calciumphosphat/Kupfer-Beschichtung nach Anspruch 1, **gekennzeichnet dadurch, dass** diese Schicht derart ausgebildet ist, dass sie Kupferionen eluiert, die die Angiogenese des angrenzenden Gewebes fördern.

4. Calciumphosphat/Kupfer-Beschichtung nach einem der vorhergehenden Ansprüche, **gekennzeichnet dadurch, dass** die Kupferkonzentration in der Beschichtung so gewählt ist, dass eine hohe Anfangselution durch die Auflösung elementaren Kupfers hinreichend antimikrobiell wirkt und moderate längerzeitige Auflösung von Kupferphosphaten keine toxische Wirkung hervorruft.

5. Calciumphosphat/Kupfer-Schicht nach einem der vorhergehenden Ansprüche, **gekennzeichnet dadurch, dass** die Schicht Kupfer in einer solchen Rate freisetzen kann, dass sich in einer das Implantat umgebenden Körperflüssigkeit eine Kupfer-Konzentration von 90 bis 160 µmol/l ergibt.

6. Calciumphosphat/Kupfer-Schicht nach einem der vorhergehenden Ansprüche, **gekennzeichnet dadurch, dass** die Schichtdicke 20±10 µm beträgt.

7. Implantat mit einer Beschichtung nach einem der vorhergehenden Ansprüche.

8. Verfahren zur Herstellung einer Beschichtung nach den Ansprüchen 1 bis 6, **gekennzeichnet durch** folgende Schritte:
- es wird ein Implantat bereitgestellt;
- das Implantat wird zunächst mit Calciumphosphat beschichtet, welches elektrochemisch abgeschieden wird;
- in die zuvor aufgebrachte Calciumphosphat-Schicht wird nachträglich Kupfer eingebracht.

9. Verfahren nach Anspruch 8, **dadurch gekennzeichnet, dass** das Calciumphosphat abgeschieden wird, insbesondere elektrochemisch, während sich das Implantat in einem Ultraschallbad befindet.

10. Verfahren nach einem der Ansprüche 8 bis 9, **dadurch gekennzeichnet, dass** das Kupfer galvanisch abgeschieden wird.

11. Verfahren nach Anspruch 10, **dadurch gekennzeichnet, dass** das Kupfer abgeschieden wird, während sich das Implantat in einem Ultraschallbad befindet.

12. Verfahren nach den Ansprüchen 9 bis 11, **dadurch gekennzeichnet, dass** das Calciumphosphat mit einer Dicke von etwa 20 µm Dicke abgeschieden wird.

13. Verfahren nach einem der Ansprüche 8 bis 12, **dadurch gekennzeichnet, dass** das Kupfer mit einer Masse von etwa 1 µg/mm² abgeschieden wird.

## Claims

1. A calcium phosphate/copper coating for an implant, **characterized in that** it comprises highly porous calcium phosphate and discontinuously distributed copper, the highly porous calcium phosphate initially forming a highly porous calcium phosphate layer in which the copper has been subsequently incorporated so as to be distributed discontinuously and inhomogeneously to form the calcium phosphate/copper coating.

2. The calcium phosphate/copper coating according to claim 1, **characterized in that** the calcium phosphate layer has a highly porous structure which allows the layer to be modified in a controlled manner both during deposition and also by a post-treatment in regard to its phase composition and solubility, and thus a controlled resorption of this layer is possible.

3. The calcium phosphate/copper coating according to claim 1, **characterized in that** said layer is configured to elute copper ions that promote the angiogenesis of the adjacent tissue.

4. The calcium phosphate/copper coating according to any of the preceding claims, **characterized in that** the copper concentration in the coating is selected such that a high initial elution by the dissolution of elemental copper has a sufficiently antimicrobial effect and a moderate longer-term dissolution of copper phosphates does not cause any toxic effect.

5. The calcium phosphate/copper coating according to any of the preceding claims, **characterized in that** the layer can release copper at such a rate that a copper concentration of 90 to 160 µmol/l is obtained in a body fluid surrounding the implant.

6. The calcium phosphate/copper coating according to any of the preceding claims, **characterized in that** the layer thickness amounts to 20±10 µm.

7. An implant comprising a coating according to any of the preceding claims.

8. A method of producing a coating according to claims 1 to 6, **characterized by** the steps of:
- providing an implant;
- initially coating the implant with calcium phosphate, which is deposited electrochemically;
- subsequently incorporating copper into the previously applied calcium phosphate layer.

9. The method according to claim 8, **characterized in that** the calcium phosphate is deposited, in particular electrochemically, while the implant is in an ultrasonic bath.

10. The method according to any of claims 8 and 9, **characterized in that** the copper is electrodeposited.

11. The method according to claim 10, **characterized in that** the copper is deposited while the implant is in an ultrasonic bath.

12. The method according to claims 9 to 11, **characterized in that** the calcium phosphate is deposited to a thickness of about 20 µm.

13. The method according to any of claims 8 to 12, **characterized in that** the copper is deposited to have a mass of about 1 µg/mm².

## Revendications

1. Revêtement de phosphate de calcium et de cuivre pour un implant, **caractérisé en ce qu'**il comprend du phosphate de calcium très poreux et du cuivre distribué de façon discontinue, le phosphate de calcium très poreux formant tout d'abord une couche de phosphate de calcium très poreuse dans laquelle le cuivre a été incorporé ultérieurement de façon discontinue et à distribution inhomogène pour former le revêtement de phosphate de calcium et de cuivre.

2. Revêtement de phosphate de calcium et de cuivre selon la revendication 1, **caractérisé en ce que** la couche de phosphate de calcium présente une structure très poreuse qui permet une modification contrôlée de la couche quant à la composition de phase et la solubilité tant pendant le dépôt que par un traitement postérieur, une résorption commandée de cette couche étant ainsi possible.

3. Revêtement de phosphate de calcium et de cuivre selon la revendication 1, **caractérisé en ce que** cette couche est réalisée de manière à éluer des ions de cuivre qui favorisent l'angiogenèse du tissu adjacent.

4. Revêtement de phosphate de calcium et de cuivre selon l'une des revendications précédentes, **caractérisé en ce que** la concentration de cuivre dans le revêtement est choisie de telle sorte qu'une élution initiale élevée par la dissolution de cuivre élémentaire a un effet suffisamment antimicrobien et une dissolution modérée à durée plus longue de phosphates de cuivre ne produit pas d'effet toxique.

5. Couche de phosphate de calcium et de cuivre selon l'une des revendications précédentes, **caractérisée en ce que** la couche est apte à libérer du cuivre à un tel taux qu'une concentration en cuivre de 90 à 160 µmol/l est obtenue dans un fluide corporel entourant l'implant.

6. Couche de phosphate de calcium et de cuivre selon l'une des revendications précédentes, **caractérisée en ce que** l'épaisseur de la couche est de 20 ± 10 µm.

7. Implant présentant un revêtement selon l'une des revendications précédentes.

8. Procédé de fabrication d'un revêtement selon les revendications 1 à 6, **caractérisé par** les étapes suivantes :
- fournir un implant ;
- tout d'abord revêtir l'implant de phosphate de calcium qui est déposé par voie électrochimique ;
- incorporer du cuivre ultérieurement dans la couche de phosphate de calcium précédemment appliquée.

9. Procédé selon la revendication 8, **caractérisé en ce que** le phosphate de calcium est déposé en particulier par voie électrochimique, pendant que l'implant se trouve dans un bain à ultrasons.

10. Procédé selon l'une des revendications 8 à 9, **caractérisé en ce que** le cuivre est déposé par galvanisation.

11. Procédé selon la revendication 10, **caractérisé en ce que** le cuivre est déposé pendant que l'implant se trouve dans un bain à ultrasons.

12. Procédé selon les revendications 9 à 11, **caractérisé en ce que** le phosphate de calcium est déposé avec une épaisseur d'environ 20 µm.

13. Procédé selon l'une des revendications 8 à 12, **caractérisé en ce que** le cuivre est déposé avec une masse d'environ 1 µg/mm².
